# EUROPEAN PATENT APPLICATION

(11) **EP 4 810 083 A1**
(43) Date of publication of application: **23.09.2026**
(21) Application number: 24891031.7
(22) Date of filing: 08.08.2024
(51) Int. Cl.: A61M 5/30, A61M 5/178

(54) **CONTAINER UNIT FOR SYRINGE AND SYRINGE**

(30) Priority: 15.11.2023 JP 2023194756
(71) Applicant: Daicel Corporation, Osaka-shi, Osaka 530-0011 (JP)
(72) Inventor: HASEGAWA, Takashi, Tokyo 108-8230 (JP)
(74) Representative: Grünecker Patent- und Rechtsanwälte PartG mbB
(86) International application number: PCT/JP2024/028560
(87) International publication number: WO 2025/104987

(57) **Abstract**

A container unit for an injector includes: a container including an accommodation space in which an injection liquid is accommodated; and a container holder to which the container is removably attachable, the container holder being used with the container attached when the injector is used, in which the container holder includes a nozzle portion having an injection port through which the injection liquid is injected when the injector is used, and a nozzle flow path through which the injection liquid is guided from the accommodation space to the injection port when the injector is used, the container includes a filling opening portion through which the accommodation space is directly filled with the injection liquid from outside in a state where the container is detached from the container holder, the filling opening portion being configured to communicate with the nozzle flow path in a state where the container is attached to the container holder, and an opening cross-sectional area of the filling opening portion is larger than an opening cross-sectional area of the injection port.

## Description

### Technical Field

The present disclosure relates to a container unit for an injector and to an injector.

### Background Art

As a device that injects a liquid medicine or the like to a target region of a living body or the like, a needle-equipped injector that injects an injection liquid to a target region via an injection needle, and a needleless injector that injects an injection liquid to a target region without using an injection needle are known. Particularly, in recent years, needleless injectors are under development, having also gained attention for their ease of handling and hygiene. In general, needleless injectors include a configuration brought into practical application, in which a liquid medicine pressurized by a driving source such as a compressed gas or a spring is injected toward a target region, and the liquid medicine is injected to the inside of the target region through use of the kinetic energy of the liquid medicine.

For example, Patent Document 1 discloses a needleless injector in which an accommodation portion for accommodating an injection liquid is formed. Furthermore, Patent Document 2 discloses an injector including an accommodation portion for accommodating an injection liquid, which is provided with a lateral hole and filled with the injection liquid.

### Citation List

### Patent Document

Patent Document 1: International Publication WO 2022/004334
Patent Document 2: International Publication WO 1994/013541

### Summary of Invention

### Technical Problem

Since an injector is usually required to inject an injection liquid from an injection port at a high pressure, the injection port is formed as a fine hole. Therefore, in a known injector, when filling an accommodation portion for accommodating an injection liquid with an injection liquid, it has been necessary to suction the injection liquid through an injection port formed as a fine hole. Particularly when the injection liquid has a high viscosity, a filling process with the injection liquid requires a considerable amount of time.

The technology of the present disclosure has been made in view of the above circumstances, and an object thereof is to provide a technology related to a container unit for an injector capable of being quickly filled with an injection liquid.

### Solution to Problem

To solve the above problems, the present disclosure adopts the following configuration in a container unit for an injector. That is, the technology of the present disclosure is a container unit for an injector, the unit including: a container including an accommodation space in which an injection liquid is accommodated; and a container holder to which the container is removably attachable, the container holder being used with the container attached when the needleless injector is used (when the needleless injector is actuated), in which the container holder includes a nozzle portion having an injection port through which an injection liquid is injected when the injector is used (when the injector is actuated) and a nozzle flow path through which the injection liquid is guided from the accommodation space to the injection port when the injector is used (when the injector is actuated), the container includes a filling opening portion through which the accommodation space is directly filled with the injection liquid from outside in a state where the container is detached from the container holder, the filling opening portion being configured to communicate with the nozzle flow path in a state where the container is attached to the container holder, and an opening cross-sectional area of the filling opening portion is larger than an opening cross-sectional area of the injection port.

In the container unit for an injector in the present disclosure, a proximal end-side opening portion formed on a proximal end side of the nozzle flow path may be disposed to face the filling opening portion in a state where the container is attached to the container holder. By arranging the proximal end-side opening portion and the filling opening portion to face each other in this manner, smooth flow of the injection liquid is ensured, and the pressurized injection liquid can be injected without being decelerated. Furthermore, the pressure applied from inside to the container and the container holder decreases, improving the durability.

In the container unit for an injector in the present disclosure, the proximal end-side opening portion of the container holder is included within a projection region onto which the filling opening portion is projected along an axial direction of the container unit in a state where the container is attached to the container holder. The proximal end-side opening portion of the container holder is included within the projection region onto which the filling opening portion is projected along the axial direction of the container unit. With this configuration, the smooth flow of the injection liquid is ensured, and the pressurized injection liquid can be injected without being decelerated. Furthermore, the pressure applied from inside to the container and the container holder decreases, improving the durability.

In the container unit for an injector in the present disclosure, the proximal end-side opening portion and the filling opening portion may be coaxially arranged in a state where the container is attached to the container holder. By coaxially arranging the proximal end-side opening portion and the filling opening portion, the smooth flow of the injection liquid is ensured, and the pressurized injection liquid can be injected without being decelerated. Furthermore, the pressure applied from inside to the container and the container holder decreases, improving the durability.

The technology of the present disclosure may be an injector including the container unit for an injector and a housing to which the container unit is removably attached. The injector may be a needleless injector or a needle-equipped injector.

### Advantageous Effects of Invention

According to the technology of the present disclosure, it is possible to provide a technology related to a container unit for an injector capable of being quickly filled with an injection liquid.

### Brief Description of Drawings

FIG. 1 is an overall view of a needleless injector according to an embodiment.
FIG. 2 is a cross-sectional view of the needleless injector.
FIG. 3 is a configuration diagram of a container unit for the needleless injector.
FIG. 4 is an enlarged view of FIG. 3, in relation to a container and container holder of the container unit.
FIG. 5 is a cross-sectional view of an assembly of the container unit and a plunger.
FIG. 6 is an explanatory view illustrating a method of filling the container unit with an injection liquid.

### Description of Embodiments

An embodiment of the present disclosure is described below with reference to the drawings. The present disclosure illustrates, as an example of an injector, a needleless injector that injects an injection liquid to a target region without using an injection needle. However, the technology of the present disclosure may be applied to a needle-equipped injector that injects the injection liquid into the target region via the injection needle. Configurations and a combination thereof in each embodiment are examples, and additions, omissions, replacements, and other changes in configuration can be made as appropriate within the scope not departing from the spirit of the present invention. The present disclosure is not limited by the embodiments, and is limited only by the claims.

### <Embodiment>

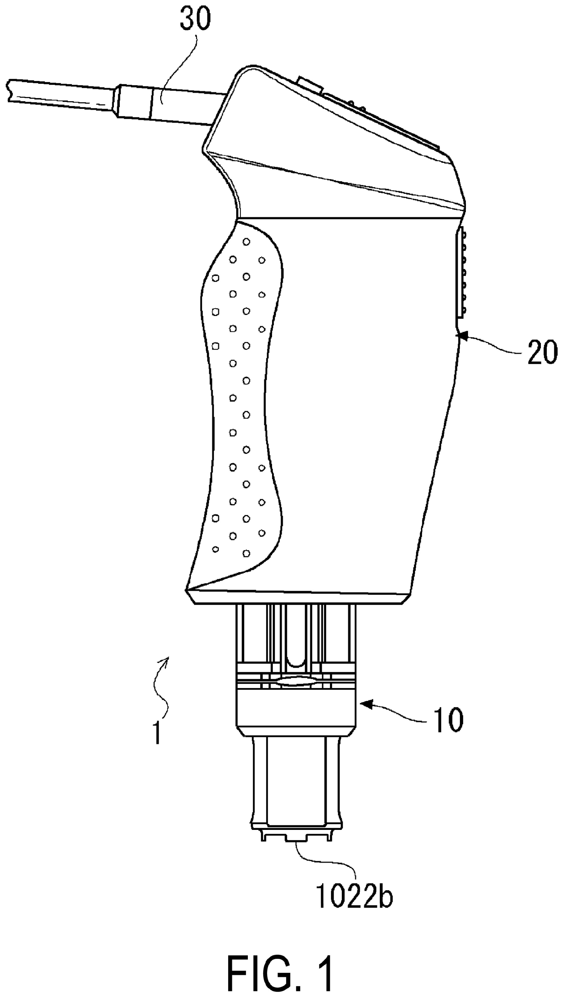

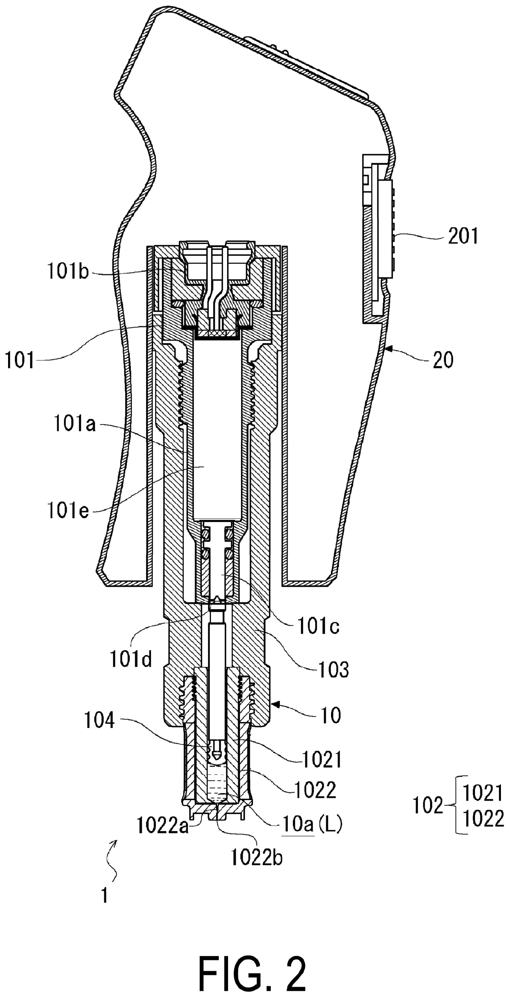

FIG. 1 is an overall view of a needleless injector 1 according to the embodiment. FIG. 2 is a cross-sectional view of the needleless injector 1. Here, the needleless injector 1 is formed with an injector assembly 10 attached to a housing 20. The needleless injector 1 is a needleless injector that injects an injection liquid L to a target region, using combustion energy of an explosive, that is, a device that performs an injection by injecting the injection liquid L to the target region without using an injection needle.

In the present embodiment, as terms indicating a relative positional relationship in a longitudinal direction of the needleless injector 1, "distal end side" and "proximal end side" are used. The term "distal end side" indicates a position close to the distal end of the needleless injector 1 to be described below, that is, a position close to an injection port 1022b. The term "proximal end side" indicates a direction opposite to the "distal end side" in the longitudinal direction of the needleless injector 1, that is, a position close to the housing 20.

Note that, in the following description in the present disclosure, the injection liquid L to be injected to the target region by the needleless injector 1 is made of a liquid medium containing a predetermined substance, which exerts an intended effect or function for the target region. In the injection liquid L, the predetermined substance may be in a state of being dissolved in a liquid as a medium, or may be in a state of being simply mixed instead of being dissolved.

Examples of the predetermined substance contained in the injection liquid L include a biologically-derived substance that can be injected to the target region that is a living body, and a substance that exhibits a desired physiological activity. For example, examples of the biologically-derived substance include DNA, RNA, a nucleic acid, an antibody, and a cell. Examples of the substance that exhibits the physiological activity include various substances exerting pharmacological or therapeutic effects, which include, for example, medicines composed of low molecular compounds, proteins, or peptides, a vaccine, an inorganic substance such as metal particles for thermotherapy or radiotherapy, and a support serving as a carrier. Furthermore, the liquid as a medium of the injection liquid L is only required to be a substance suitable for administering these predetermined substances into the target region, regardless of whether the liquid is aqueous or oleaginous. In addition, the viscosity of the liquid as the medium is not particularly limited as long as the predetermined substance can be injected by the needleless injector 1.

Furthermore, the target region that is an injection target of the injection liquid L is a region to which the injection liquid L is to be administered, and may include, for example, a cell or tissue of an organism (skin or the like), and an organ (an eyeball, a heart, or a liver). Note that, within a range that does not cause any problems, an organism component in a state of being separated from an organism body can be set as the target region. That is, not only in-vivo injection of the predetermined substance to the target region, but also ex-vivo injection of the predetermined substance to the target region (a tissue or an organ) and in-vitro injection of the predetermined substance to the target region (a cultured cell or a cultured tissue) are included within an operation range of the injector according to the present embodiment.

### [Overall Structure]

As illustrated in FIG. 1, the needleless injector 1 includes the injector assembly 10, the housing 20 to which the injector assembly 10 can be attached, and a power cable 30 for supplying a drive current to the injector assembly 10 in the housing 20. In the needleless injector 1, the injector assembly 10 is configured to be removably attached to the housing 20. As illustrated in FIG. 2, the injector assembly 10 is an assembly including an actuator 101, a container unit 102, an attachment 103, and a plunger 104. In the injector assembly 10, an accommodation space 10a formed in a container 1021 to be described later is filled with the injection liquid L according to a filling method with the injection liquid to be described later in a preparation stage before use (before actuation) of the needleless injector 1. The injector assembly 10 is a unit that is replaced every time the injection liquid L is injected. That is, when the needleless injector 1 is used, the injector assembly 10 including the actuator 101 and the container unit 102 is connected to the housing 20 and actuated, and after the needleless injector 1 is used, the injector assembly 10 is removed from the housing 20 and replaced.

The actuator 101 is a member that generates energy for the needleless injector 1 to inject the injection liquid L. As illustrated in FIG. 2, the actuator 101 includes a body 101a, an initiator 101b, and a piston 101c. The actuator 101 uses the initiator 101b as an actuation source, and uses the piston 101c as an output portion. The body 101a is formed in a tubular shape, and an opening portion 101d is formed at a distal end of the body 101a. The initiator 101b is an electric igniter that releases a combustion product by combusting an ignition agent, and is fitted in the body 101a and thus closes a proximal end portion of the body 101a. The piston 101c is disposed between the initiator 101b and the opening portion 101d, and can slide inside the body 101a in a longitudinal direction. A space between the initiator 101b and the piston 101c forms a combustion chamber 101e in which the combustion product is released.

Here, examples of the ignition agent for use in the initiator 101b include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and ferric oxide (AFO), and an explosive composed of a combination of two or more of these explosives. These explosives exhibit characteristics that, although the explosives generate high-temperature and high-pressure plasma during combustion immediately after ignition, when the combustion product condenses at room temperature, the explosives do not contain gaseous components and accordingly the pressure generated decreases abruptly. An explosive other than these explosives may be used as the ignition agent as long as appropriate injection of the injection liquid L can be performed.

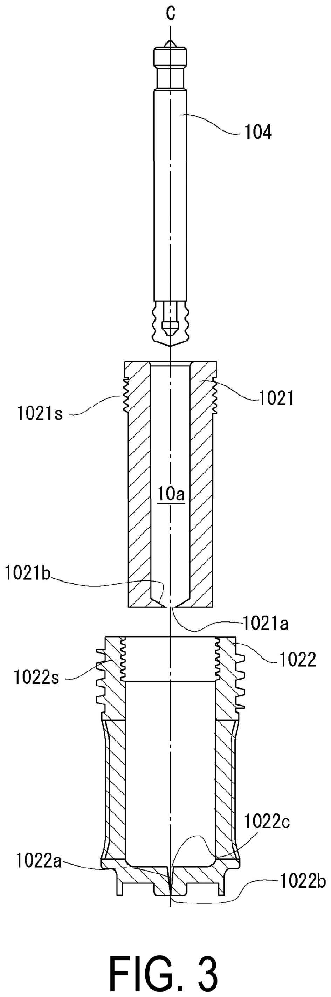

The container unit 102 is a member for accommodating the injection liquid L. The container unit 102 includes the container 1021 and a container holder 1022. FIG. 3 is a configuration diagram of the container unit 102 of the needleless injector 1. The container 1021 can be removably attached to the container holder 1022. Both the container 1021 and the container holder 1022 are formed in a substantially cylindrical shape. When the container 1021 is attached to the container holder 1022, the center axis of the container 1021 and the center axis of the container holder 1022 are coaxially arranged. The accommodation space 10a for accommodating the injection liquid L is formed in the container 1021. In addition, a nozzle portion 1022a having a truncated cone-shaped nozzle flow path 1022f is formed in the container holder 1022. An injection port 1022b is formed at the distal end of the nozzle portion 1022a, and is formed through which the injection liquid L can be injected. The container unit 102 will be described in detail later.

The attachment 103 is a member that couples the body 101a and the container unit 102. The attachment 103 is formed in a substantially cylindrical shape, with the actuator 101 fitted in the internal space of the attachment 103 on its proximal end side and with the container holder 1022 threadedly engaged with the attachment 103 on its distal end side. The inner circumferential surface of the attachment 103 and the outer circumferential surface of the body 101a are threadedly engaged with each other, and the inner circumferential surface of the attachment 103 and the outer circumferential surface of the container holder 1022 are threadedly engaged with each other, whereby the body 101a and the container unit 102 are coupled via the attachment 103.

The plunger 104 is a member that pressurizes the injection liquid L by the energy received from the actuator 101. The plunger 104 is accommodated in the attachment 103 and is disposed between the actuator 101 and a filling opening portion 1021a of the container 1021. The plunger 104 is formed in a rod shape, and a proximal end portion thereof is engaged with a distal end portion of the piston 101c. Furthermore, a distal end portion of the plunger 104 is inserted into the accommodation space 10a formed inside the container 1021. As illustrated in FIG. 3, the container 1021, the container holder 1022, and the plunger 104 are formed such that when assembled, the center axis of the plunger 104, the center axis of the container 1021, and the center axis of the container holder 1022 are coaxial. In the present embodiment, all the center axes are collectively referred to as a center axis C.

The housing 20 is a member that accommodates the injector assembly 10 and functions as a grip portion that a user grasps to use the needleless injector 1. The outer surface of the housing 20 is provided with a plurality of switches including a switch 201 for operating the needleless injector 1 to perform the injection of the injection liquid L. The plurality of switches are connected to a control unit (not illustrated) such as a microcomputer incorporated in the housing 20. The inner surface of the housing 20 is provided with a socket (not illustrated) connected to the initiator 101b of the injector assembly 10. Power is supplied to the control unit and the initiator 101b via the power cable 30 connected to the housing 20. The control unit controls supply of a drive current to the initiator 101b in response to a signal from each switch, thereby controlling the operation of the needleless injector 1.

When the drive current is supplied to the initiator 101b by the user's operation and the initiator 101b is activated, the combustion product is released from the initiator 101b to the combustion chamber 101e. When the pressure in the combustion chamber 101e increases, the piston 101c receives the pressure and slides toward the distal end side of the body 101a. Consequently, the plunger 104 engaged with the piston 101c is pushed toward the distal end side, and injection energy is applied to the injection liquid L accommodated in the accommodation space 10a. Thus, the injection liquid L is injected from the injection port 1022b formed at the distal end of the nozzle portion 1022a. The kinetic energy of the injection liquid L ruptures the surface of the target region such as skin, and the injection liquid L is injected into the target region.

### [Container Unit]

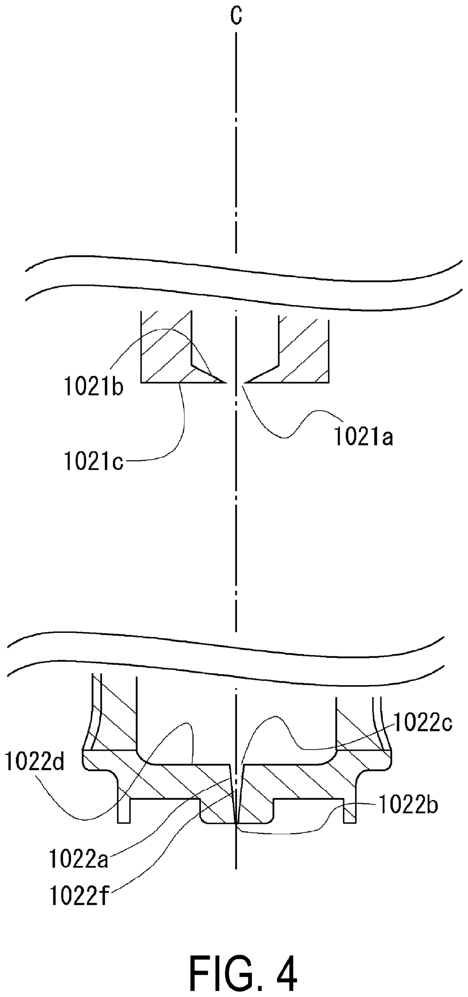

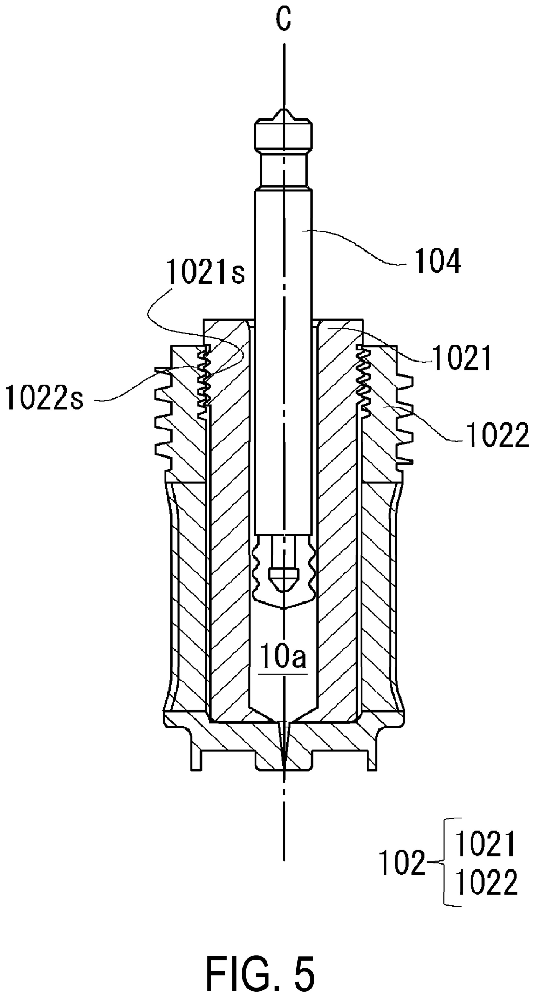

FIG. 4 is an enlarged view of FIG. 3 in relation to the container 1021 and the container holder 1022 of the container unit 102. FIG. 5 is a cross-sectional view of an assembly of the container unit 102 and the plunger 104. The container unit 102 will be described below in detail with reference to FIGS. 3, 4, and 5.

The container 1021 is formed in, for example, a substantially cylindrical shape. The filling opening portion 1021a is formed at the distal end of the accommodation space 10a formed to be columnar inside the container 1021. Although details will be described later, the filling opening portion 1021a is an opening portion to be used when the accommodation space 10a of the container 1021 is filled with the injection liquid L. Furthermore, a taper 1021b is formed on the distal end side of the accommodation space 10a with its diameter decreasing toward the filling opening portion 1021a. Threads 1021s are formed on the outer circumferential surface of the container 1021 to be threadedly engaged with the container holder 1022. The filling opening portion 1021a is formed as, for example, a circular through hole formed around the center axis C. By arranging the filling opening portion 1021a on the center axis C, the smooth flow of the injection liquid L is ensured, and the pressurized injection liquid L can be injected without being decelerated. Furthermore, the pressure applied from inside to the container 1021 and the container holder 1022 decreases, improving the durability. The filling opening portion 1021a is formed through which the injection liquid L can be supplied from the accommodation space 10a to the nozzle flow path 1022f to be described later during use (during actuation) of the needleless injector 1, and is formed through which the accommodation space 10a can be directly filled with the injection liquid L in a state where the container 1021 is detached from the container holder 1022. A method of filling the accommodation space 10a with the injection liquid L will be described later. The taper 1021b is formed at the distal end of the accommodation space 10a with a diameter decreasing from the proximal end side to the distal end side, and is formed as an inclined wall surface for smoothly guiding the injection liquid L or air bubbles in the accommodation space 10a to the nozzle portion 1022a to be described later. However, the shape of the container 1021 is not limited to the cylindrical shape, and may be appropriately changed in accordance with the shape of the housing 20 or the attachment 103. The presence or absence of the taper 1021b, or the form of the taper 1021b may be appropriately changed. Furthermore, the shape of the opening cross section of the filling opening portion 1021a is not limited to a circle, and may be a triangle, a quadrangle, or the like. For example, in a case where the opening cross section of the filling opening portion 1021a has a shape with a corner, when the container 1021 is filled with the injection liquid L, a portion of a tool used for the filling (for example, a pipette tip attached to a micropipette) is in contact with sides in the vicinity of the corner at two points and can thus be supported. This allows the distal end of the tool to be held in a stable posture. As a result, the filling process with the injection liquid L can be stably performed, thereby contributing to a reduction in air bubble formation during filling.

The container holder 1022 is formed in, for example, a substantially cylindrical shape. Inside the container holder 1022 formed in the cylindrical shape, an internal space is formed into which the container 1021 can be fitted, and threads 1022s are formed on a wall portion of the internal space, allowing the container 1021 to be threadedly engaged. Accordingly, as illustrated in FIG. 5, in the container unit 102, the container 1021 and the container holder 1022 can be removably attached to each other by engaging the threads 1022s formed on the inner circumferential surface of the container holder 1022 and the threads 1022s formed on an outer circumferential surface of the container 1021. Furthermore, threads that are engageable with the attachment 103 are formed on the outer circumferential surface of the container holder 1022. At the distal end of the container holder 1022, the nozzle portion 1022a is formed including the nozzle flow path 1022f communicating with the accommodation space 10a formed in the container 1021 via the filling opening portion 1021a when the container 1021 is attached to the container holder 1022. The injection port 1022b, which is a circular through hole formed around the center axis C, is formed at the distal end of the nozzle portion 1022a. On the proximal end side, a proximal end-side opening portion 1022c is formed, which is a circular through hole formed around the center axis C and allows the injection liquid L to flow into the injection port 1022b. Although details will be described later, during actuation of the needleless injector 1 (initiator 101b), the injection liquid L contained in the container 1021 flows into the nozzle flow path 1022f of the container holder 1022 through the filling opening portion 1021a, and is injected from the injection port 1022b at a high pressure. In the present embodiment, a cross-sectional shape of the filling opening portion 1021a may be the same as that of the proximal end-side opening portion 1022c. This configuration has an advantage that the injection liquid L can be smoothly guided.

As illustrated in FIG. 4, in the nozzle portion 1022a, the truncated cone-shaped nozzle flow path 1022f is formed with its diameter decreasing to gradually reduce the opening cross-sectional area from the proximal end-side opening portion 1022c toward the injection port 1022b. The opening cross-sectional area of the nozzle flow path 1022f is a cross-sectional area in a direction orthogonal to the center axis C of the needleless injector 1. The nozzle flow path 1022f is a space with its proximal end side defined by the proximal end-side opening portion 1022c and its distal end side defined by the injection port 1022b. The nozzle flow path 1022f is formed as a flow path for the injection liquid L, and thus the injection liquid L flows from the accommodation space 10a into the injection port 1022b during use (during actuation) of the needleless injector 1. By changing the shape of the nozzle flow path, the injection pressure of the injection liquid L to be injected from the injection port 1022b can be adjusted.

When the container 1021 is attached to the container holder 1022, the center axes of the container holder 1022 and the container 1021 are coaxially arranged, and thus the proximal end-side opening portion 1022c and the filling opening portion 1021a are coaxially arranged. The proximal end-side opening portion 1022c and the filling opening portion 1021a are arranged to face each other. Accordingly, during use (during actuation) of the needleless injector 1, the injection liquid L accommodated in the accommodation space 10a is supplied to the nozzle flow path 1022f through the filling opening portion 1021a and the proximal end-side opening portion 1022c, and the injection liquid L is injected from the injection port 1022b.

The shape of the container holder 1022 is not particularly limited, and may be changed as appropriate in accordance with the shape of the housing 20 or the attachment 103. The shape of the opening cross section of the proximal end-side opening portion 1022c, and the shape of the opening cross section of the injection port 1022b are not limited to a circle, and may be a triangle, a quadrangle, or the like.

The opening cross-sectional area of the filling opening portion 1021a is formed to be larger than the opening cross-sectional area of the injection port 1022b. The opening cross-sectional area described herein is a cross-sectional area in the direction orthogonal to the center axis C of the needleless injector 1. The opening cross-sectional area of the filling opening portion 1021a may be formed to be larger than the opening cross-sectional area of the proximal end-side opening portion 1022c. Since the opening cross-sectional area of the filling opening portion 1021a is formed to be larger than the opening cross-sectional area of the proximal end-side opening portion 1022c, the smooth flow of the injection liquid L is ensured, and the pressurized injection liquid L can be injected without being decelerated. Furthermore, since the smooth flow of the pressurized injection liquid L is ensured as described above, the pressure applied from inside to the container 1021 and the container holder 1022 can be decreased, and the durability thereof can be improved. In the present embodiment, both the filling opening portion 1021a and the proximal end-side opening portion 1022c are formed in a circular shape, and formed to allow the injection liquid L to pass therethrough. In the present embodiment, the filling opening portion 1021a may be formed to have an opening diameter of 0.8 mm to 4.85 mm, and the proximal end-side opening portion 1022c may be formed to have an opening diameter of 0.8 mm. The opening cross-sectional area of the injection port 1022b is designed to have an appropriate size depending on the designed injection pressure of the injection liquid L injected from the injection port 1022b during actuation of the needleless injector 1 (initiator 101b), and may have an opening diameter of, for example, about 0.1 mm. In this manner, by forming the opening cross-sectional area of the filling opening portion 1021a to be larger than that of the injection port 1022b, it is possible to easily fill the accommodation space 10a with the injection liquid L when filling the space with the injection liquid L to be described later. However, the shape and opening diameter of the filling opening portion 1021a and the proximal end-side opening portion 1022c described above are merely examples, and are not limited thereto.

In addition, in the container unit 102 in the present embodiment, the nozzle flow path 1022f of the container holder 1022 is included within a projection region onto which the filling opening portion 1021a is projected in a direction along the center axis C of the container unit 102 in a state where the container 1021 is attached to the container holder 1022. That is, the proximal end-side opening portion 1022c of the container holder 1022 is included within the projection region of the filling opening portion 1021a described above. Accordingly, in the state where the container 1021 is attached to the container holder 1022, it is possible to prevent the proximal end-side opening portion 1022c of the container holder 1022 from being closed by an edge portion forming the filling opening portion 1021a in the container 1021. Accordingly, during use (during actuation) of the needleless injector 1, the injection liquid L can be smoothly guided from the accommodation space 10a to the nozzle flow path 1022f.

Reference sign 1021c illustrated in FIG. 4 denotes an outer surface formed on the distal end side of the container 1021 (hereinafter referred to as "distal end abutment outer surface"). Reference sign 1022d denotes an inner surface formed on the distal end side of the container holder 1022 (hereinafter referred to as "distal end abutment inner surface"). For example, in the state where the container 1021 is attached to the container holder 1022, the distal end abutment outer surface 1021c of the container 1021 and the distal end abutment inner surface 1022d of the container holder 1022 are in a state of abutting each other. In the container unit 102 according to the present embodiment, a sealing material such as an O-ring or a packing may be disposed between the distal end abutment outer surface 1021c of the container 1021 and the distal end abutment inner surface 1022d of the container holder 1022. Accordingly, a region between the distal end abutment outer surface 1021c of the container 1021 and the distal end abutment inner surface 1022d of the container holder 1022 can be sealed. As a result, it is possible to prevent the injection liquid L from leaking from the region between the distal end abutment outer surface 1021c and the distal end abutment inner surface 1022d. The sealing material described above may be provided on either the distal end abutment outer surface 1021c or the distal end abutment inner surface 1022d. For example, an annular recess may be provided around the proximal end-side opening portion 1022c on the distal end abutment inner surface 1022d of the container holder 1022, and a sealing member such as an O-ring may be disposed in the recess. For another example, an annular recess may be provided around the filling opening portion 1021a on the distal end abutment outer surface 1021c of the container 1021, and a sealing material such as an O-ring may be disposed in the recess. However, the form of a groove for disposing the sealing material and the form of the sealing material are not particularly limited.

### [Filling Method with Injection Liquid]

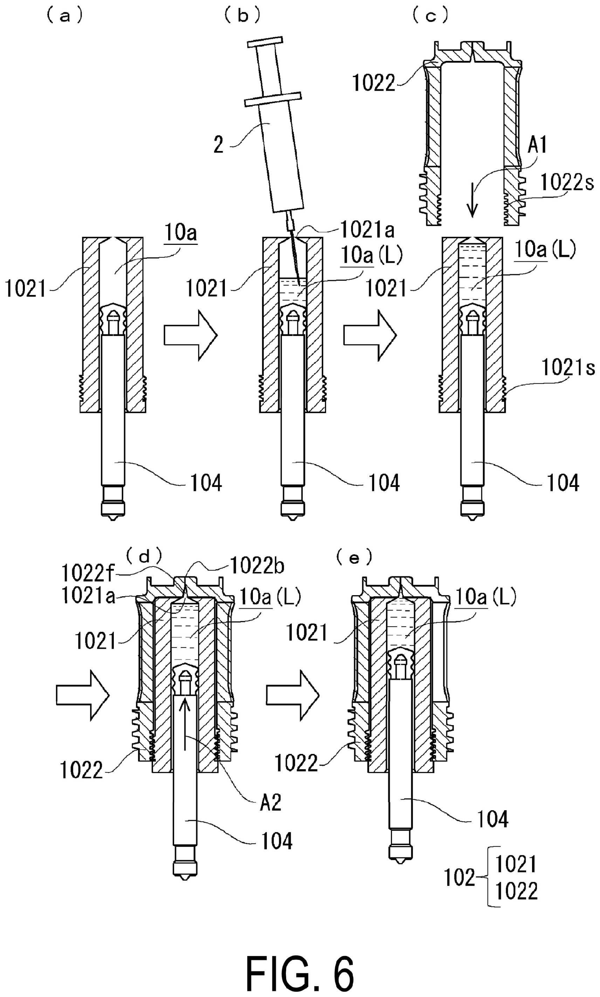

FIG. 6 is an explanatory view illustrating a method of filling the container unit 102 (container 1021) with the injection liquid L. In each of FIGS. 6(a) to 6(e), the distal end side of the container unit 102 (container 1021) is illustrated as the upper side of the drawing, and the proximal end side of the container unit 102 (container 1021) is illustrated as the lower side of the drawing. A process of filling the accommodation space 10a of the container 1021 with the injection liquid L is typically performed while the filling opening portion 1021a formed on the distal end side of the container 1021 is directed upward. Hereinafter, a method for filling the accommodation space 10a of the container 1021 with the injection liquid L, and a method for assembling the container unit 102 and the plunger 104, which is a subsequent process, will be described in five steps with reference to FIGS. 6(a) to 6(e).

FIG. 6(a) is an explanatory view of step S1, and illustrates a state where the plunger 104 is inserted into the container 1021. By inserting the plunger 104 into the container 1021, an opening formed on a rear end side of the container 1021 is closed, and the container 1021 is ready for filling the accommodation space 10a with the injection liquid L.

FIG. 6(b) is an explanatory view of step S2, and illustrates a state where the accommodation space 10a is filled with the injection liquid L using a filling tool 2 such as a micropipette or an injector. The opening cross-sectional area of the filling opening portion 1021a of the container 1021 has a size into which the distal end portion of the filling tool 2 can be inserted, for example. For example, the size of the opening cross-sectional area of the filling opening portion 1021a is designed from the viewpoint of the filling ability (filling efficiency) of the injection liquid L in the accommodation space 10a, and is designed regardless of the injection pressure of the injection liquid L injected from the nozzle portion 1022a when the needleless injector 1 is used (when the initiator 101b is actuated). In the present embodiment, the accommodation space 10a can be directly filled with the injection liquid L from outside in a state where the distal end portion of the filling tool 2 is inserted into the filling opening portion 1021a, and thus the filling process with the injection liquid L can be efficiently and easily performed in a short time. Therefore, even if the injection liquid L to be filled into the accommodation space 10a of the container 1021 has a high viscosity, the injection liquid L can still be easily filled in a short time. The filling tool 2 described above is not limited to the micropipette or the injector, and may be another type of tool.

FIG. 6(c) is an explanatory view of step S3, and illustrates a state where the container holder 1022 is fitted into the container 1021 in a direction of arrow A1. The container holder 1022 is fitted into the container 1021, and the threads 1021s and the threads 1022s are assembled and engaged with each other, whereby the container 1021 is attached to the container holder 1022, and the container unit 102 is completed. In this state, the filling opening portion 1021a of the container 1021 and the nozzle flow path 1022f (including the injection port 1022b and the proximal end-side opening portion 1022c) in the container unit 102 are coaxially arranged.

FIG. 6(d) is an explanatory view of step S4, and illustrates a state where the plunger 104 inserted into the container 1021 is pushed in a direction of arrow A2. When the accommodation space 10a is filled with the injection liquid L in step S2, a gap may be formed between the liquid surface of the injection liquid L and the filling opening portion 1021a. In this case, the plunger 104 is pushed in by a predetermined amount to fill the gap with the injection liquid L. As a result, the injection liquid L filled into the accommodation space 10a is pressurized, and a small amount of injection liquid L is injected from the injection port 1022b. In this case, air bubbles in the accommodation space 10a can be discharged together with the small amount of injection liquid L from the injection port 1022b to the outside. Accordingly, the accommodation space 10a and the nozzle flow path 1022f can be filled with the injection liquid L without containing air bubbles. In this manner, by injecting a small amount of injection liquid L from the injection port 1022b with the distal end side of the container unit 102 directed upward and the proximal end side thereof directed downward, it is possible to easily remove air bubbles contained in the injection liquid L and air present in the flow path for the injection liquid L.

FIG. 6(e) is an explanatory view of step S5, and illustrates a state where a process of filling the container 1021 with the injection liquid L and a process of assembling the container unit 102 are completed. In this state, the container unit 102 and the plunger 104 can be attached to the attachment 103. Thereafter, the threads provided on the outer circumferential surface of the container holder 1022 are engaged with the threads provided in the internal space of the attachment 103, whereby the coupling of the container unit 102 and the attachment 103 is completed.

### [Actions and Effects]

As described above, the container unit 102 of the needleless injector 1 according to the present embodiment includes the container 1021 including the accommodation space 10a for accommodating the injection liquid L, and the container holder 1022 to which the container 1021 is removably attachable, the container holder 1022 being used with the container 1021 attached when the needleless injector 1 is used (when the needleless injector is actuated), in which the container holder 1022 includes the nozzle portion 1022a having the injection port 1022b through which the injection liquid L is injected when the needleless injector 1 is used (when the injector is actuated), and the nozzle flow path 1022f through which the injection liquid L is guided from the accommodation space 10a to the injection port 1022b when the needleless injector 1 is used (when the injector is actuated), the container 1021 includes the filling opening portion 1021a through which the accommodation space 10a is directly filled with the injection liquid L from outside in the state where the container 1021 is detached from the container holder 1022, the filling opening portion 1021a is configured to communicate with the nozzle flow path 1022f in a state where the container 1021 is attached to the container holder 1022, and the opening cross-sectional area of the filling opening portion 1021a is larger than the opening cross-sectional area of the injection port 1022b.

Specifically, the container unit 102 in the present embodiment adopts a structure in which the nozzle portion 1022a having the injection port 1022b is not formed on the container 1021 side, but is formed on the container holder 1022 side, which is removably attachable to the container 1021, and the filling opening portion 1021a is formed in the container 1021. An opening (injection port 1022b) for injecting the injection liquid L toward outside at a high pressure when using (actuating) the needleless injector 1, and an opening (filling opening portion 1021a) for filling the accommodation space 10a of the container 1021 with the injection liquid L are separately formed. In this way, since the filling opening portion 1021a on the container 1021 side, and the injection port 1022b on the container holder 1022 side are separately configured, the opening cross-sectional areas of the filling opening portion 1021a and the injection port 1022b can be individually and optimally designed depending on their respective required functions and specifications. That is, typically, the opening cross-sectional area on the order of microns is required for the injection port 1022b through which the injection liquid L is injected toward outside at a high pressure when the needleless injector 1 is used (when the needleless injector is actuated). In a related art, it has been necessary to suction the injection liquid through a fine-hole nozzle even when filling a container with the injection liquid, and therefore a process of filling the container with the injection liquid requires time. This disadvantage becomes particularly noticeable when the viscosity of the injection liquid is high. In contrast, in the container unit 102 of the present embodiment, the size of the filling opening portion 1021a can be designed independently regardless of the injection pressure of the injection liquid L injected through the injection port 1022b when the needleless injector 1 (initiator 101b) is actuated. That is, the size of the filling opening portion 1021a can be optimally designed with a focus on the filling ability (filling efficiency) of the injection liquid L in the accommodation space 10a. In a state where the container 1021 is detached from the container holder 1022, the accommodation space 10a is filled with the injection liquid L through the filling opening portion 1021a optimally designed as described above, and thus the accommodation space 10a can be easily filled with the injection liquid L in a short time.

In the needleless injector 1 according to the present embodiment, when the accommodation space 10a is filled with the injection liquid L, the filling tool 2 is used with the filling opening portion 1021a of the container 1021 directed upward, thereby directly filling the accommodation space 10a with the injection liquid L, and thus, any gap into which air enters is not generated between the injection liquid L and the distal end portion of the plunger 104, thus suppressing the generation of air bubbles. Even if air bubbles are generated when the accommodation space 10a is filled with the injection liquid L, the injection port 1022b is located above when the container 1021 is attached to the container holder 1022, and thus, the air bubbles can be easily and quickly removed by pressurizing the injection liquid L filled in the accommodation space 10a by the plunger 104 after waiting for the air bubbles to rise.

In particular, in the needleless injector 1 of the present embodiment, the proximal end-side opening portion 1022c of the nozzle portion 1022a is disposed to face the filling opening portion 1021a in a state where the container 1021 is attached to the container holder 1022, the proximal end-side opening portion 1022c of the container holder 1022 is included within a projection region onto which the filling opening portion 1021a is projected along the axial direction of the container unit 102 in a state where the container 1021 is attached to the container holder 1022, the proximal end-side opening portion 1022c and the filling opening portion 1021a are coaxially arranged in a state where the container 1021 is attached to the container holder 1022, and the size of the opening cross-sectional area of the filling opening portion 1021a is equal to or greater than that of the opening cross-sectional area of the proximal end-side opening portion 1022c. According to these configurations, even if the filling opening portion 1021a on the container 1021 side, and the injection port 1022b on the container holder 1022 side are separately configured depending on their respective specifications, the injection liquid L can be appropriately injected through the injection port 1022b without closing the flow path for the injection liquid L when the needleless injector 1 is used (when the needleless injector is actuated).

In the container unit 102 of the present embodiment, the proximal end-side opening portion 1022c in the nozzle flow path 1022f is disposed to face the filling opening portion 1021a in a state where the container 1021 is attached to the container holder 1022. Consequently, the smooth flow of the pressurized injection liquid L can be ensured, and the pressurized injection liquid L can be injected without being decelerated. Furthermore, by ensuring the smooth flow of the pressurized injection liquid L, the pressure applied from inside to the container 1021 and the container holder 1022 can be decreased, and the durability of the container unit 102 and the needleless injector 1 is improved. Furthermore, the container unit 102 of the present embodiment has a structure in which the proximal end-side opening portion 1022c and the filling opening portion 1021a are arranged to face each other, and thus even if air bubbles are generated when the accommodation space 10a is filled with the injection liquid L, the air bubbles can be easily and quickly removed from the injection port 1022b by pressurizing the injection liquid L.

In the container unit 102 of the present embodiment, the proximal end-side opening portion 1022c of the container holder 1022 is included within a projection region onto which the filling opening portion 1021a is projected along the axial direction of the container unit 102 in a state where the container 1021 is attached to the container holder 1022. Consequently, the smooth flow of the injection liquid L pressurized during use (during actuation) of the needleless injector 1 can be ensured, and the pressurized injection liquid L can be injected without being decelerated. Furthermore, by ensuring the smooth flow of the pressurized injection liquid L, the pressure applied from inside to the container 1021 and the container holder 1022 can be decreased, and the durability of the container unit 102 is improved.

In the container unit 102 of the present embodiment, the proximal end-side opening portion 1022c and the filling opening portion 1021a are coaxially arranged in a state where the container 1021 is attached to the container holder 1022. The proximal end-side opening portion 1022c and the filling opening portion 1021a are arranged on the center axis C. Consequently, the smooth flow of the injection liquid L pressurized during use (during actuation) of the needleless injector 1 can be ensured, and the pressurized injection liquid L can be injected without being decelerated. Furthermore, by ensuring the smooth flow of the pressurized injection liquid L, the pressure applied from inside to the container 1021 and the container holder 1022 can be decreased, and the durability of the container unit 102 is improved.

### <Application to Needle-Equipped Injector>

As described above, the container unit 102 according to the present embodiment may be applied to a needle-equipped injector. For example, a mode may be adopted in which an injection needle can be attached to the injection port 1022b in the container unit 102 described above, and the injection liquid L is injected through the injector needle. As a structure for fixing the injection needle to the nozzle portion 1022a, a screw type, a fitting type, or any suitable structure may be adopted. In an aspect in which the container unit 102 is applied to the needle-equipped injector, the injection liquid L pressurized during use (during actuation) of the needle-equipped injector reaches the injection port 1022b through the nozzle flow path 1022f, and then flows into the flow path formed inside the injection needle. The injection liquid L supplied to the injection needle at high pressure in this manner is injected into a target region from the injection port formed at the distal end of the injection needle. In a case where the container unit 102 according to the present embodiment is applied to the needle-equipped injector, the plunger 104 may be manually driven to apply injection energy to the injection liquid L accommodated in the accommodation space 10a. In the aspect in which the container unit 102 according to the present embodiment is applied to the needle-equipped injector, the same technical effects as those in the case where the container unit 102 is applied to the needleless injector 1 are obtained.

Each aspect disclosed in the present specification can be combined with any other feature disclosed in the present specification.

### Reference Signs List

1: Needleless injector
2: Filling tool
20: Housing
30: Power cable
201: Switch
10: Injector assembly
101: Actuator
101a: Body
102: Container unit
1021: Container
1022: Container holder
103: Attachment
104: Plunger

## Claims

1. A container unit for an injector, the unit comprising:
a container including an accommodation space in which an injection liquid is accommodated; and
a container holder to which the container is removably attachable, the container holder being used with the container attached when the injector is used, wherein
the container holder includes a nozzle portion having an injection port through which the injection liquid is injected when the injector is used, and a nozzle flow path through which the injection liquid is guided from the accommodation space to the injection port when the injector is used,
the container includes a filling opening portion through which the accommodation space is directly filled with the injection liquid from outside in a state where the container is detached from the container holder, the filling opening portion being configured to communicate with the nozzle flow path in a state where the container is attached to the container holder, and
an opening cross-sectional area of the filling opening portion is larger than an opening cross-sectional area of the injection port.

2. The container unit according to claim 1, wherein a proximal end-side opening portion formed on a proximal end side of the nozzle flow path is disposed to face the filling opening portion in a state where the container is attached to the container holder.

3. The container unit according to claim 2, wherein the proximal end-side opening portion of the container holder is included within a projection region onto which the filling opening portion is projected along an axial direction of the container unit in a state where the container is attached to the container holder.

4. The container unit according to claim 2, wherein the proximal end-side opening portion and the filling opening portion are coaxially arranged in a state where the container is attached to the container holder.

5. The container unit according to claim 2, wherein a size of the opening cross-sectional area of the filling opening portion is equal to or greater than that of the opening cross-sectional area of the proximal end-side opening portion.

6. An injector comprising:
the container unit according to any one of claims 1 to 5; and
a housing to which the container unit is removably attached.

7. The injector according to claim 6, wherein the injector is a needleless injector.
